# EUROPEAN PATENT SPECIFICATION

(11) **EP 2 405 952 B1**
(45) Date of publication and mention of the grant of the patent: **24.12.2014**
(21) Application number: 10709265.2
(22) Date of filing: 10.03.2010
(51) Int. Cl.: A61L 27/14, A61L 27/50, A61L 27/58

(54) **SCAFFOLDS**
GERÜSTE
ÉCHAFAUDAGES

(30) Priority: 11.03.2009 GB 0904174
(43) Date of publication of application: 18.01.2012
(73) Proprietor: The University Of Sheffield, Sheffield S10 2TN (GB)
(72) Inventor: MACNEIL, Sheila, Sheffield S7 1BP (GB); RYAN, Anthony, Sheffield S7 1RX (GB)
(74) Representative: HGF Limited
(86) International application number: PCT/GB2010/050427
(87) International publication number: WO 2010/103324

(56) References cited:
- WO-A2-2008/008266
- US-A1- 2008 220 054
- US-A1- 2009 053 287

## Description

The present invention relates generally to wound dressings comprising an electrospun scaffolds and a nonsteroidal anti-inflammatory drug.

### BACKGROUND

The incidence of chronic wounds is increasing with an ageing population in the western world. The number of patients with chronic leg ulcers, the majority of which are associated with venous disease, is estimated to be between 1 and 2 per 1000 population (At any one time 20% of people with an active leg ulceration will have an open ulcer). Additionally the incidence of diabetes is increasing and as many as 10-20% of these patients will develop non-healing diabetic foot ulcers. There are also patients poorly mobile in hospital at risk of developing bed sores. For all of these patients new approaches to accelerate healing and reduce pain are badly needed.

Pain is a major problem for the majority of patients with chronic ulcers (the exceptions are patients with diabetes and spinal injuries do not experience wound associated pain because of nerve damage). The majority of chronic ulcers are managed in the community and from the point of view of the patient the day-to day problems are twofold- pain on dressings changes and the failure of ulcers to heal. Patients with chronic wounds often experience pain on dressing changes (Noonan and Burge, Plebology. 1998;3:14-9, Price et al, Int Wound J. 2008 Jun;5(2):159-71) and the removal of the dressing itself can be associated with an inflammatory reaction. The reasons why chronic wounds fail to heal are several and interrelated - wounds are often poorly vascularised and hypoxic, commonly inflamed, often infected and as a consequence aggressive and resistant to healing.

A certain level of inflammation can accelerate wound healing as the release of pro-inflammatory cytokines attracts cells into the wound area. However, too high a level of inflammation can retard wound healing particularly in many aggressive chronic wounds where months and even years of matrix metalloprotease activity and secretion of pro-inflammatory cytokines create wound bed environments in which the normal skin cells at the perimeter of the wound fail to survive (Werner and Grose, Physiol. Rev. 2003;83:835-870, Diegelmann and Evans, Frontiers in Bioscience. 2004;9:283-289). Thus cells capable of wound healing can fail to move into aggressive wound beds as the extracellular matrix present is under constant degradation due to matrix metalloproteases (Armstrong and Jude, Journal of the American Podiatric Medical Association 2002;92(1):12-18).

The introduction of a synthetic scaffold of fibres capable of supporting normal skin cell attachment, migration and proliferation can help to accelerate wound healing by providing an immediate alternative substrate for unaffected skin cells at the wound margins to migrate across.

Pain relief in wound healing is positive for the patient in a number of ways as pain promotes psychological stress in patients while pain relief allows patients to bear compression therapy, which is often beneficial in chronic wound healing (Christian et al., Neuroimmunomodulation. 2006;13(5-6):337-46) (Price et al, Int Wound J. 2007 Apr;4 Suppl 1:1-3 and Int Wound J. 2008 Jun;5(2):159-71). Unfortunately, systemic pain relief has not proven very efficient in tackling chronic wound pain as the pain relief from most medications lasts only minutes or hours and there are concerns about patients becoming addicted to painkillers (Cardenas and Jensen, J Spinal Cord Med. 2006;29(2):109-17).

Dressings designed to reduce pain and accelerate healing were first introduced in 1948 (Gilje, Acta Derm Venereol. 1948;28(5):454-67). The dressings permitted a more rapid re-epithelialisation by reducing scab formation, and reduced the occurrence dressings sticking to the wounds and causing reinjury upon dressing removal (Hinman and Maibach, Nature. 1963 Oct 26;200:377-8). However, these dressings still required to be removed. There remains a need for an improved wound dressing which is able to reduce pain whilst promoting wound healing.

### BRIEF SUMMARY OF THE DISCLOSURE

In a first aspect the invention provides a wound dressing comprising an electrospun scaffold, characterized in that said scaffold comprises a biodegradable polymer or co-polymer and a nonsteroidal anti-inflammatory drug, wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. Preferably, said biodegradable polymer or co-polymer is co-electrospun together with said nonsteroidal anti-inflammatory drug. Said nonsteroidal anti-inflammatory drug is capable of catalyzing the degradation of said polymer or copolymer.

Said biodegradable polymer or co-polymer is biocompatible. More specifically, said biodegradable polymer is a collagen, a poly alpha ester, a polyorthoester or a polyanhydride or a copolymer thereof.

Preferably, said polymer is polylactate acid. Alternatively polymer is polyglycolic acid. Alternatively, said copolymer is a copolymer of polylactate acid and polyglycolic acid. More preferably said copolymer is a poly(D,L-lactic-co-glycolide). Still more preferably, said poly(D,L-lactic-co-glycolide) has a ratio of 75:25 lactide to glycolide. Alternatively, said poly(D,L-lactic-co-glycolide) has a ratio of 50:50 lactide to glycolide.

In a preferred embodiment, said non-steroidal anti-inflammatory drug (NSAID) is a propionic NSAID wherein said NSAID is [2-(4-Isobutylphenyl)propionic acid]. Alternatively said NSAID is acetylsalicylic acid.

Preferably, said scaffold comprises from about 5% to about 30% by weight of NSAID. Alternatively, said scaffold comprises from about 10% to 20% by weight of NSAID. Still more preferably, said scaffold comprises about 10% by weight of NSAID.

Said polymer or copolymer and said NSAID are capable of each dissolving in a common solvent.

In a further aspect, the invention provides an electrospun scaffold, said scaffold comprising a biodegradable polymer or co-polymer and a nonsteroidal anti-inflammatory drug wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. Preferably, said biodegradable polymer or co-polymer is co-electrospun together with said nonsteroidal anti-inflammatory drug. Said nonsteroidal anti-inflammatory drug is capable of catalyzing the degradation of said polymer or copolymer.
In a further aspect, the invention provides an electrospun scaffold, said scaffold comprising a biodegradable polymer or co-polymer and a nonsteroidal anti-inflammatory drug, for use as a medicament wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. Preferably, said biodegradable polymer or co-polymer is co-electrospun together with said nonsteroidal anti-inflammatory drug. Said nonsteroidal anti-inflammatory drug is capable of catalyzing the degradation of said polymer or copolymer.
In a still further aspect the invention provides an electrospun scaffold, said scaffold comprising a biodegradable polymer or co-polymer and a nonsteroidal anti-inflammatory drug, for use in the treatment of a wound wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. Preferably, said biodegradable polymer or co-polymer is co-electrospun together with said nonsteroidal anti-inflammatory drug. Said nonsteroidal anti-inflammatory drug is capable of catalyzing the degradation of said polymer or copolymer.

Preferably, said wound is a chronic wound or wherein said wound is an acute wound.

In a further aspect the invention provides an electrospun scaffold comprising a biodegradable polymer or co-polymer and a nonsteroidal anti-inflammatory drug, for use in the treatment of pain or inflammation, wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. Preferably, said biodegradable polymer or co-polymer is co-electrospun together with said nonsteroidal anti-inflammatory drug. Said nonsteroidal anti-inflammatory drug is capable of catalyzing the degradation of said polymer or copolymer.

Said biodegradable polymer or co-polymer is biocompatible.

More specifically, said biodegradable polymer is a collagen, a poly alpha ester, a polyorthoester or a polyanhydride or a copolymer thereof.

Preferably said polymer is polylactate acid. Alternatively said polymer is polyglycolic acid. Alternatively, said copolymer is a copolymer of polylactate acid and polyglycolic acid.

Preferably said copolymer is a poly(D,L-lactic-co-glycolide).Preferably, said poly(D,L-lactic-co-glycolide) has a ratio of 75:25 lactide to glycolide. Alternatively, said poly(D,L-lactic-co-glycolide) has a ratio of 50:50 lactide to glycolide.

Preferably said NSAID is a propionic NSAID.Preferably, said NSAID is [2-(4-Isobutylphenyl)propionic acid].Alternatively, said NSAID is acetylsalicylic acid.

Preferably, said scaffold comprises from about 5% to about 30% by weight of NSAID.
Still more preferably said scaffold comprises about 10% by weight of NSAID.

Said polymer or copolymer and said NSAID are capable of each dissolving in a common solvent.

Also disclosed is a method of treating a wound comprising the use of a wound dressing or an electrospun scaffold according to any one of the aforementioned embodiments.

In a further aspect, the invention provides a method of producing an electrospun scaffold for use in wound healing comprising:
i) dissolving a polymer or co-polymer and a NSAID in a common solvent wherein said NSAID is capable of catalyzing the degradation of the polymer or copolymer, and wherein: a) said biodegradable polymer is a collagen, a poly alpha ester, a polyorthoester or a polyanhydride or a copolymer thereof, and b) said NSAID is acetylsalicylic acid or a propionic NSAID;; and
ii) electrospinning the resulting solution of step i).

In a further aspect, the invention provides an electrospun scaffold produced in accordance with the method described in the aforementioned embodiment. In a further aspect the electrospun scaffold produced in accordance with the method described in the aforementioned embodiment is for use as a medicament, or for use in the treatment of a wound, such as a chronic wound or wherein said wound is an acute wound. Alternatively, the electrospun scaffold produced in accordance with the method described in the aforementioned embodiment is for use in the treatment of pain or inflammation.

In a further aspect the invention provides use of a nonsteroidal anti-inflammatory drug to catalyse the degradation of an electrospun scaffold comprising a polymer or co-polymer, wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. Preferably, said nonsteroidal anti-inflammatory drug is electrospun together with said polymer or copolymer.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

### BRIEF DESCRIPTION OF THE DRAWINGS

The invention will be described, by way of example only, with reference to the following figures.

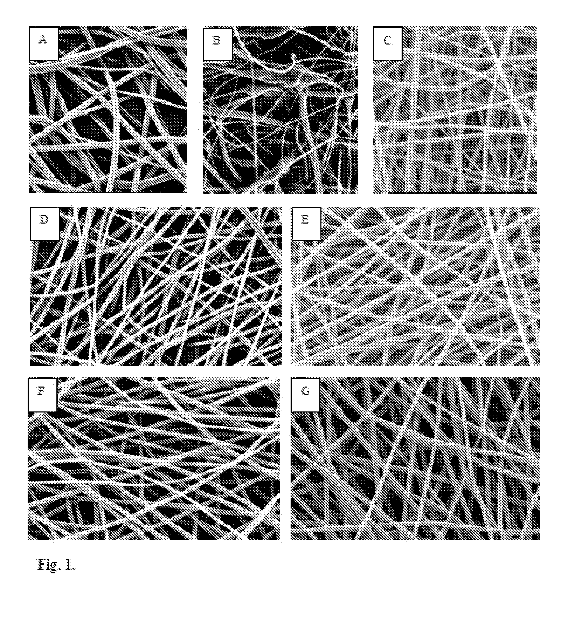

**Fig. 1****. SEM images of electrospun PLGA fibres.** A) 50/50 PLGA with 10% wt Ibuprofen; B) 50/50 PLGA with 10% wt Ibuprofen sodium salt; C) 50/50 PLGA without Ibuprofen; D) 1:1 blend of 50/50 and 75/25 PLGA with 10% wt Ibuprofen; E) 1:1 blend of 50/50 and 75/25 PLGA without Ibuprofen; F) 75/25 PLGA with 10% wt Ibuprofen; G) 75/25 PLGA without Ibuprofen. Scale bar = 50 µm.

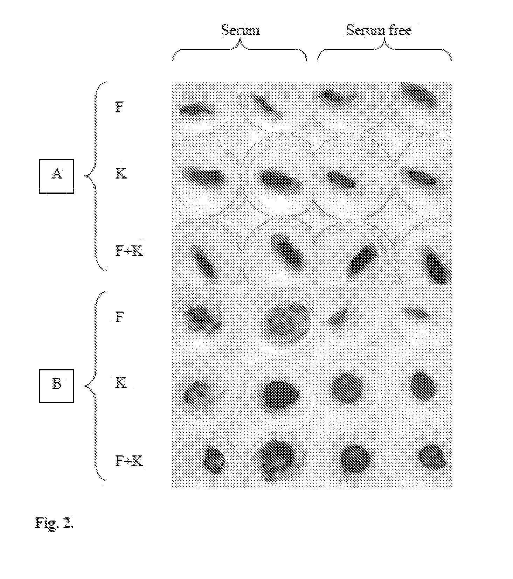

**Figure 2****. Effect of environmental conditions on electrospun PLGA 75:25 scaffolds.** Sirius red staining of collagen deposition of human fibroblasts (F), keratinocytes (K) and co-cultures of both (F+K) grown for 1 week (in serum or serum free conditions) in 75:25 PLGA scaffolds spun in different conditions. A) Scaffolds were spun in high humidity (>85%); B) As for A, but then immediately after spinning, scaffolds were dried under high vacuum at room temperature for 24 hours.

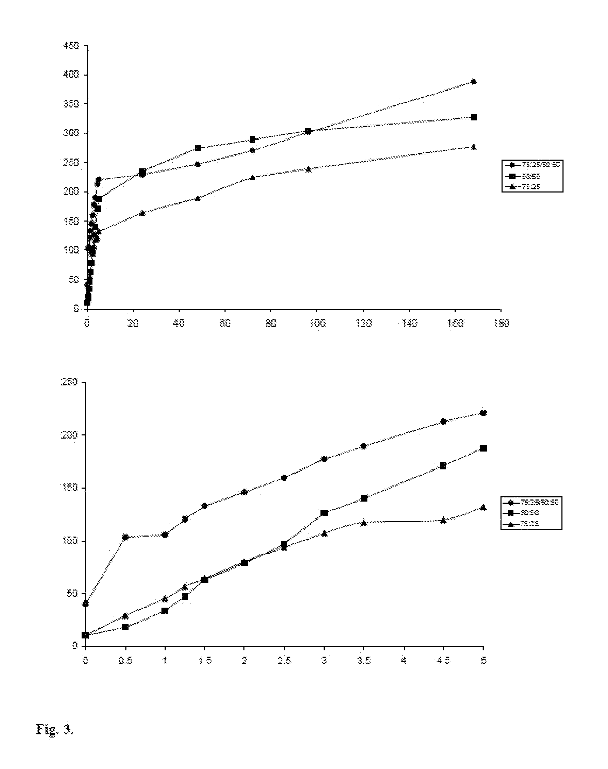

Figure 3. Ibuprofen release profiles for three PLGA scaffold types: 75/25 (o), 50/50 (x) and a 1:1 blend of these two (+). The concentrations of Ibuprofen were measured by UV/Vis spectroscopy at 222 nm at various time points for one week.

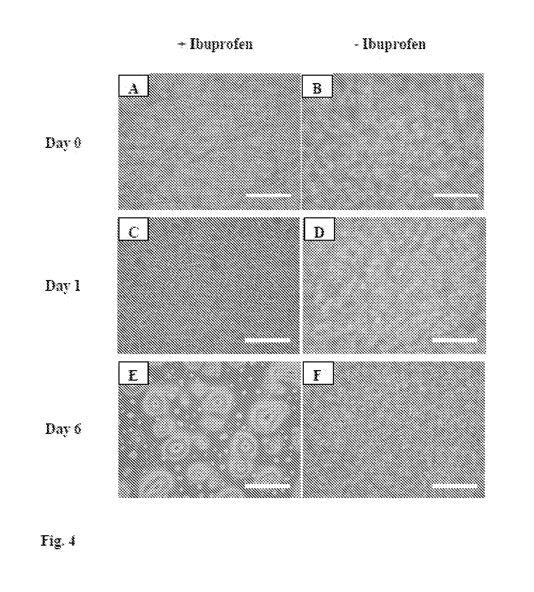

**Figure. 4****. *In vitro* degradation of PLGA 75:25 scaffold in Ringers solution over time.** Optical micrographs of scaffolds with 10% wt Ibuprofen on the right hand side A, C and E at day 0, 1 and 6 of incubation in Ringers solution. Scaffolds without Ibuprofen on the left hand side B, D and F. Scale bar= 100 mm

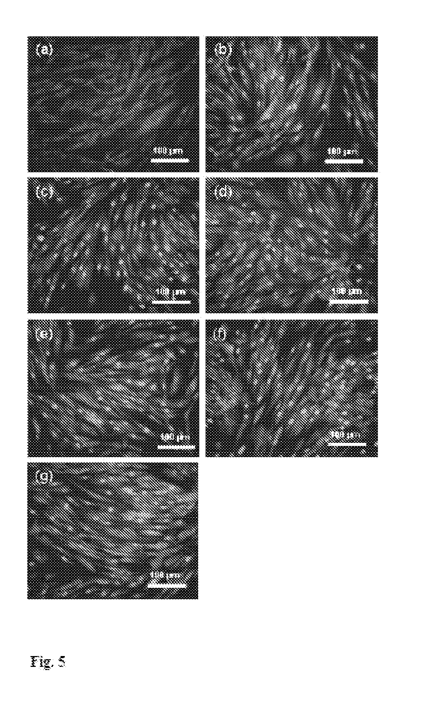

**Figure 5****. NFkB immunolabelling of 2D HDF cultures after treatment with TNF-a, LPS and Ibuprofen.** Immunofluorescent labelling of human fibroblasts for the p65 subunit of NF-κB, demonstrating relative transcription factor activity. (a) Control unstimulated cells. (b) LPS (100 ngml⁻¹) stimulated cells. (c) TNF-α (1000 Uml⁻¹) stimulated cells. (d) LPS (100 ngml⁻¹) plus Ibuprofen (10⁻³ M) stimulated cells. (e) TNF-α (1000 Uml⁻¹) and Ibuprofen (10⁻³ M) stimulated cells. (f) LPS (100 ngml⁻¹) and Ibuprofen sodium salt (10⁻⁴ M) stimulated cells. (g) TNF-α (1000 Uml⁻¹) and Ibuprofen sodium salt (10⁻⁴ M) stimulated cells.

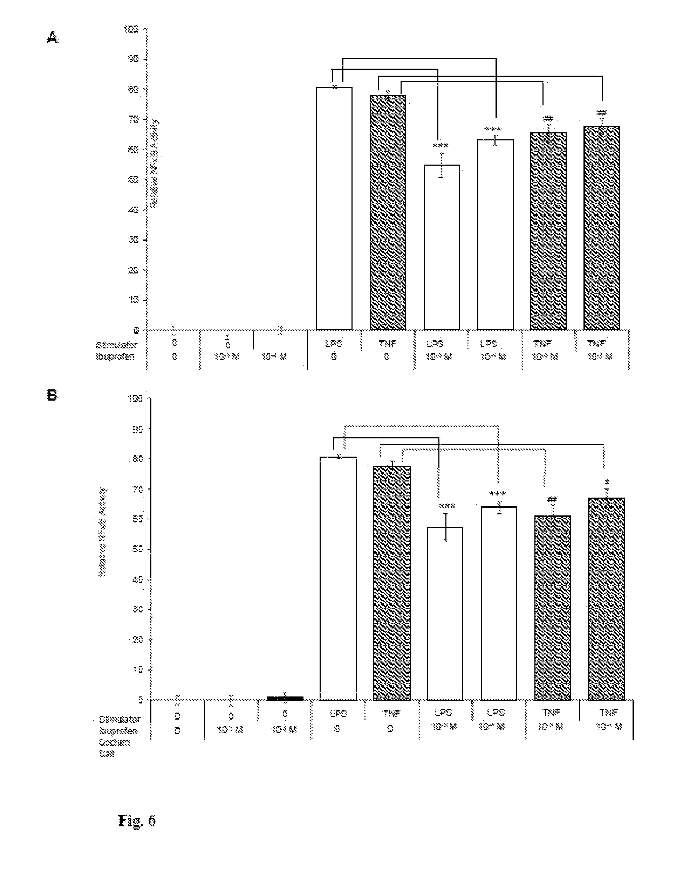

**Figure 6****. Anti-inflammatory effect of Ibuprofen on HDF cells assessed by NF-kB immunolabelling**. Effect of (a) Ibuprofen and (b) Ibuprofen sodium salt on NFkB inflammatory signalling. Human fibroblasts were incubated with media or Ibuprofen compounds (10⁻⁴ to 10⁻³ M) for 15 minutes before stimulation with LPS (100 ngml⁻¹, open bars) or TNF-α (1000 Uml⁻¹, diagonal striped bars), or Ibuprofen compounds alone (filled bars) for 90 minutes. Inflammatory signalling was determined using intracellular localisation of NF-κB. Key: *** p < 0.001 compared to LPS alone, # p < 0.05, ## p < 0.001 compared to TNF-α alone.

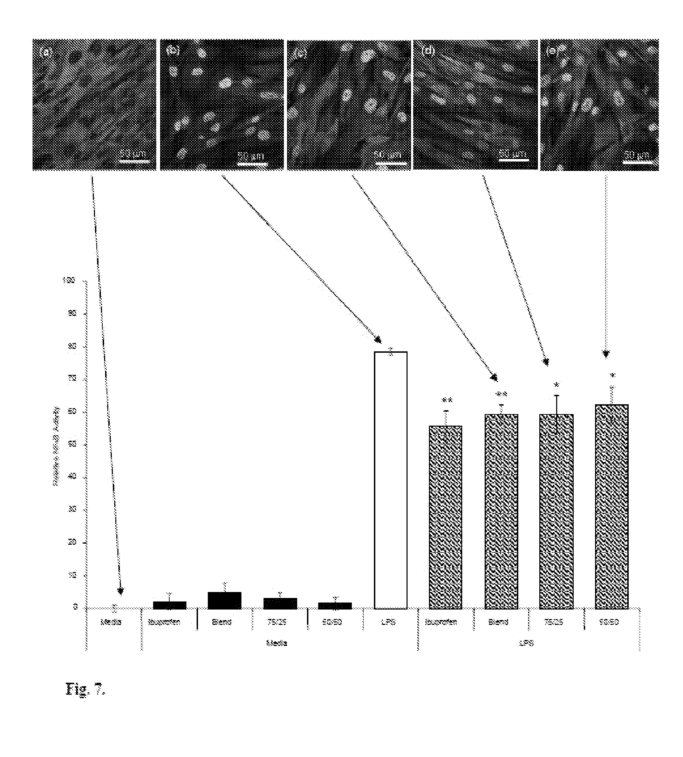

**Figure 7****. Effect of Ibuprofen loaded scaffolds on TNF-a and LPS stimulated NFkB activation in fibroblasts.**
   Nuclear translocation of the proinflammatory transcription factor NFkB was examined as illustrated in photos a-e in A and quantified in B for 2D cultures of fibroblasts exposed to Ibuprofen releasing scaffolds.
   In A (a) control unstimulated fibroblasts are shown (b) shows LPS (100 ngml-1) stimulated fibroblasts (2h exposure) (c) shows cells exposed to LPS (100 ngml-1) for 2h after pre-incubation with PLA/PGA Ibuprofen scaffolds for 24 h. In c the scaffold was a 50/50 blend of 75/25 and 50/50 PLA/PGA loaded with Ibuprofen (d) shows LPS (100 ngml⁻¹) stimulated fibroblasts exposed to 75/25 PLA/PGA loaded with Ibuprofen and (e) shows LPS (100 ngml-1) stimulated fibroblasts exposed to 50/50 PLA/PGA loaded with Ibuprofen.
   Quantification of nuclear translocation of NFkB is shown for these fibroblasts in the histograms beneath in B for cells under non-stimulated conditions (where the percentage of NFkB in the nucleus did not exceed 5 %) and for cells stimulated with LPS (100 ngml⁻¹) for 2h after pre-incubation with PLA/PGA ibuprofen scaffolds for 24 h. Results shown are means + SEM of triplicate cultures and statistical significance is indicated as * p < 0.05, ** p < 0.001.

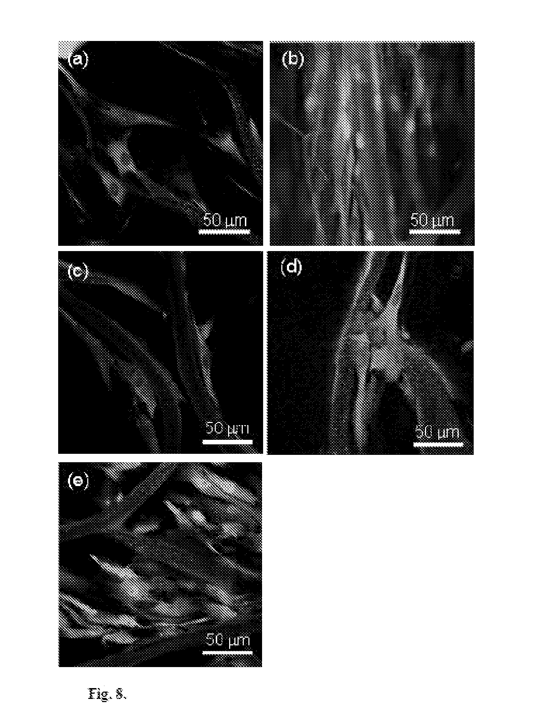

**Figure 8****. NFkB immunolabelling of 3D HDF cultures after treatment with LPS and Ibuprofen scaffolds.** Fibroblasts were cultured in a 3D Azowipe scaffolds (details as in Materials and Methods) and exposed to Ibuprofen loaded scaffolds. Immunofluorescent labelling of cells for the p65 subunit of NF-κB was used to demonstrate the relative transcription factor activity. (a) Control unstimulated cells. (b) HDF stimulated with LPS (100 ngml⁻¹) for 2h. (c) HDF stimulated with LPS (100 ngml⁻¹) for 2h after pre-incubation with a 50/50 blend of 75/25 and 50/50 PLA/PGA Ibuprofen scaffolds for 24 h. (d) HDF stimulated with LPS (100 ngml⁻¹) for 2h after pre-incubation with 75/25 PLA/PGA Ibuprofen scaffolds (e) HDF stimulated with LPS (100 ngml⁻¹) for 2h after pre-incubation with 50/50 PLA/PGA Ibuprofen scaffolds.

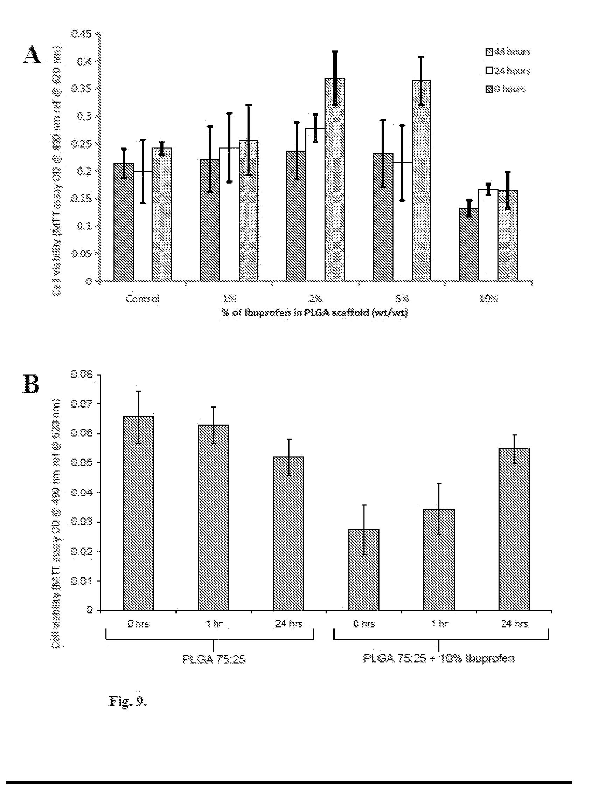

**Figure 9****. Culture of fibroblasts on Ibuprofen loaded scaffolds**. Fibroblasts were cultured on control and Ibuprofen loaded PLGA 75:25 scaffolds (details as in Materials and Methods) for 48hr (A) or 7 days (B) and their survival assessed by MTT-ESTA assay. In A scaffolds were loaded with 0, 1,2 5 or 10% Ibuprofen and then washed by immersion in media for 0,24 or 48 hrs before cells were cultured on them. In B scaffolds were either unloaded or loaded with 10% Ibuprofen and then washed for 0, 1 or 24 hrs prior to cell culture. Results shown are means + SD of triplicate wells of cells.

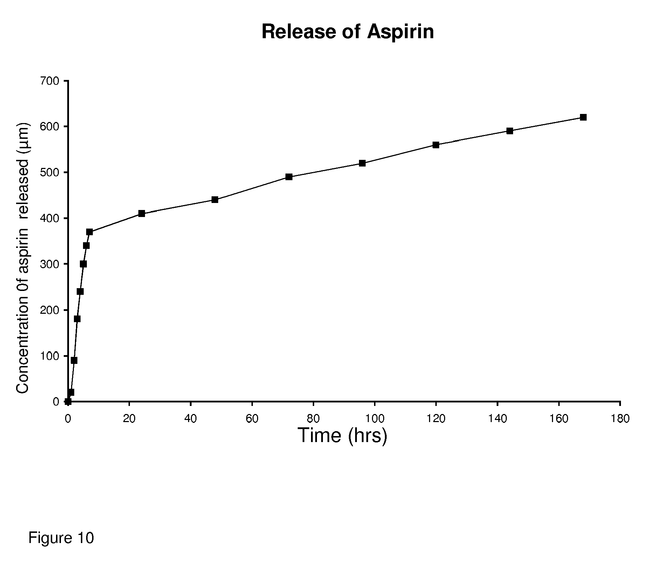

**Figure 10****. Release of Aspirin from PLGA electrospun scaffolds.**
   Aspirin release profile from PLGA scaffold types: 75/25. The concentration of Aspirin released from the fibres was monitored using UV/Vis spectroscopy (intensity at 240 nm) at various time points over a one week period and compared with calibration curve values.

### DETAILED DESCRIPTION

The present invention provides a wound dressing comprising an electrospun scaffold, the scaffold comprising a biodegradable polymer or co-polymer and a nonsteroidal anti-inflammatory drug (NSAID) wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. The inventors have surprisingly identified that NSAIDs are capable of catalyzing the degradation of the polymer or copolymer and thus the breakdown of a scaffold, making these loaded scaffolds particularly useful in wound healing and overcoming difficulties relating to dressing removal.

The wound dressing initially releases the NSAID whilst simultaneously acting as a temporary tissue guide for skin regeneration. The wound dressing can degrade into the wound bed leaving skin cells in place so there is no need to remove the dressing from the wound.

The level of released NSAID is sufficient to reduce the response of cells to major pro-inflammatory cytokines without compromising the basic ability of the scaffolds to act as substitute dermal scaffolds for the attachment and migration of human dermal fibroblasts.

The inventors have demonstrated the loading of Ibuprofen into scaffolds which then release the Ibuprofen over several days. The levels of Ibuprofen being released are sufficient to reduce the response of cells to major pro-inflammatory cytokines without compromising the basic ability of the scaffolds to act as substitute dermal scaffolds for the attachment and migration of human dermal fibroblasts.

To load Ibuprofen into scaffolds the acid form of Ibuprofen was dissolved in the same solvent as the PLGA polymer (DCM) and this gave homogeneous distribution of ibuprofen through the scaffold fibres. In contrast the sodium salts did not dissolve in DCM and produced very irregular scaffolds in which the release of drug was much less predictable. Thus drugs which can be dissolved in the organic solvent of the polymer can be directly spun into the scaffold fibres.

The inventors have shown that approximately 10% of the Ibuprofen loaded was released from the scaffold fibres over several days and that the incorporation of Ibuprofen accelerated fibre breakdown. The concentration of Ibuprofen being released was shown to be sufficient to attenuate the inflammatory response of fibroblasts to a major pro-inflammatory cytokine, TNF-α, and to lipopolysaccharide which would normally be derived from bacteria.

The scaffolds releasing Ibuprofen still retained the ability to act as scaffolds for skin cell attachment, migration and proliferation. This was confirmed by looking at the attachment and proliferation of human dermal fibroblasts on the scaffolds. Results showed that while cell attachment to the freshly prepared Ibuprofen loaded scaffolds was reduced at the highest concentration of Ibuprofen loaded (10%) this was restored once scaffolds had sat in PBS for 24 hours after sterilisation, when cell attachment increased back to levels seen in the control unloaded scaffolds. This shows that the Ibuprofen loading into the scaffolds does not change their basic ability to act as fibres for skin cell attachment and migration and indicates that these fibres may be used as tissue guides for wound healing applications.

With respect to wound healing a certain level of inflammation accelerates wound healing as the release of pro-inflammatory cytokines attracts cells into the wound area - the converse is also true and too high a level of inflammation can retard wound healing (Werner and Grose Physiol. Rev. 2003;83:835-870, Diegelmann and Evans, Frontiers in Bioscience2004;9:283-289. ). This is certainly thought to be the case in many aggressive chronic wounds where months and even years of matrix metalloprotease activity and secretion of pro-inflammatory cytokines create wound bed environments in which the normal skin cells at the perimeter of the wound fail to survive if they migrate into the wound bed. Thus cells capable of wound healing fail to move into these aggressive wound beds as the extracellular matrix present is under constant degradation due to matrix metalloproteases (e.g. Armstrong and Jude, Journal of the American Podiatric Medical Association 2002;92(1):12-18).

The introduction of a synthetic scaffold of fibres capable of supporting normal skin cell attachment and migration and proliferation will help to accelerate wound healing by providing an immediate alternative substrate for unaffected skin cells at the wound margins to migrate across. The simultaneous release of an anti-inflammatory agent from these fibres will serve to reduce the aggressive inflammation seen in these aggressive inflamed wound beds.

Wound healing involves the steps of clot formation, plugging the wound, invasion of the clot by inflammatory cells, fibroblasts and capillaries, drawing the wound margins together, followed by migration forward of the epidermal wound edges to cover the wound surface by re-epithelialisation.

As used herein, the term "wound" refers to, but is not limited to: injuries or trauma to internal or external tissue, preferably injury or trauma to the epidermis and/or dermis of the skin. The wound may be an acute wound or a chronic wound. By way of example, an acute wound may be an incision, laceration, abrasion graze or burn, a puncture wound, a penetration wound or a wound due to dermatologic diseases such as psoriasis, acne and eczema. By way of example, a chronic wound may be a venous ulcer, a diabetic ulcer, a pressure ulcer, corneal ulcer, digestive ulcer or wounds as a result of ischemia and radiation poisoning.

It is common practice in wound treatment to cover a wound in a wound dressing. As used here in, the term "wound dressing" refers to a dressing for application to a wound, wherein said dressing facilitates the healing process of the wound by providing an electrospun scaffold surface that acts as a tissue guide for wound repair and re-epithelialization. The electrospun scaffold comprises a biodegradable polymer or co-polymer and a nonsteroidal anti-inflammatory drug, wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID.

Preferably a wound dressing will be capable of attachment to the wound site. It should also be non-toxic and elicit no more than a minimal allergenic response. In addition the dressing should prevent bacteria or viral infections from entering the wound. Examples of wound dressings of the invention relates to any structure suitable for application to a wound characterised by the presence of electrospun scaffold for application to a wound comprising a biodegradable polymer or co-polymer and a nonsteroidal anti-inflammatory drug, wherein said polymer or copolymer and said NSAID are capable of each dissolving in a common solvent and said NSAID is capable of catalyzing the degradation of the polymer or copolymer, wherein: a) said biodegradable polymer is a collagen, a poly alpha ester, a polyorthoester or a polyanhydride or a copolymer thereof, and b) said NSAID is acetylsalicylic acid or a propionic NSAID. By way of example only, the dressing may be prosthesis, implant, matrix, stent, cell culture dishes, gauze, bandage, plaster, biodegradable matrix and polymeric film.

The term "scaffold", as used herein, refers to any material that allows attachment of cells, preferably attachment of cells involved in wound healing. "Attachment", "attach" or "attaches" as used herein, refers to cells that adhere directly or indirectly to a substrate as well as to cells that adhere to other cells.

Preferably the scaffold is three dimensional.

Suitable scaffolds include meshes, other filamentous structures, non-woven, sponges, woven or non-woven materials, knit or non-knit materials, felts, salt eluted porous materials, moulded porous materials, 3D-printing generated scaffolds, foams, perforated sheets, grids, parallel fibres with other fibres crossing at various degrees, and combinations thereof. The core scaffold can be in a variety of shapes including sheets, cylinders, tubes, spheres or beads. The core scaffold may be fabricated from absorbable or non-absorbable materials.

Scaffolds may be formed of fibres of polymer, woven or non-woven into meshes that can be used to support cell attachment and growth. These scaffolds can be formed by casting, weaving, salt leaching, spinning, or moulding.

The scaffold comprises an electrospun scaffold. The terms "electrospinning" or "electrospun," as used herein refers to any method where materials are streamed, sprayed, sputtered, dripped, or otherwise transported in the presence of an electric field. The electrospun material can be deposited from the direction of a charged container towards a grounded target, or from a grounded container in the direction of a charged target. In particular, the term "electrospinning" means a process in which fibres are formed from a charged solution comprising at least one natural biological material, at least one synthetic polymer material, or a combination thereof by streaming the electrically charged solution through an opening or orifice towards a grounded target.
As used herein, the terms "solution" and "fluid" refers to a liquid that is capable of being charged and which comprises at least one natural material, at least one synthetic polymer, or a combination thereof.

The electrospun polymer may be a co-polymer. The term "co-polymer" as used herein is intended to encompass co-polymers, ter- polymers, and higher order multiple polymer compositions formed by block, graph or random combination of polymeric components.

The properties of the electrospun materials can be adjusted in accordance with the needs and specifications of the cells to be suspended and grown within them. The porosity, for instance, can be varied in accordance with the method of making the electrospun materials matrix.

As used herein, the term a natural biological material can be a naturally occurring organic material including any material naturally found in the body of a mammal, plant, or other organism. A synthetic polymer material can be any material prepared through a method of artificial synthesis, processing, or manufacture. Preferably the polymer is a biologically compatible material. Both the biological and polymeric materials are capable of being charged under an electric field.

Suitable naturally occurring materials include, but are not limited to, amino acids, polypeptides, denatured peptides such as gelatin from denatured collagen, carbohydrates, lipids, nucleic acids, glycoproteins, lipoproteins, glycolipids, glycosaminoglycans, and proteoglycans. In one embodiment the naturally occurring material is an extracellular matrix material, for example collagen, fibrin, elastin, laminin, fibronectin, heparin. Such extracellular matrix material may be isolated from cells, such as mammalian cells, for example of human origin. Preferably the naturally occurring material is collagen.

Preferably the scaffold comprises a biocompatible synthetic polymer. Examples of biocompatible synthetic polymers include, but are not limited to, poly(urethanes), poly(siloxanes) or silicones, poly(ethylene), polyvinyl pyrrolidone), poly(2-hydroxy ethyl methacrylate), poly(N-vinyl pyrrolidone), poly(methyl methacrylate), polyvinyl alcohol) (PVA), poly(acrylic acid), polyvinyl acetate), polyacrylamide, poly(ethylene-co-vinyl acetate), poly(ethylene glycol), poly(methacrylic acid), polylactic acid (PLA), polyglycolic acids (PGA), poly(lactide-co-glycolides) (PLGA), nylons, polyamides, polyanhydrides, poly(ethylene-co-vinyl alcohol) (EVOH), polycaprolactone, poly(vinyl acetate), polyvinylhydroxide, poly(ethylene oxide) (PEO) and polyorthoesters Preferably, the biocompatible polymer is PGLA.

The invention includes scaffolds comprising a combination of synthetic polymers and naturally occurring biological material, for example a combination of collagen and PLGA. The relative amounts of the synthetic polymers and naturally occurring biological material in the matrix can be tailored to specific applications

There are many factors involved in the electrospinning process which may effect scaffolds fibre diameter and pore size. The key variables are solution viscosity, surface tension, and viscoelasticity of the spinning solution. These are directly related to the concentration of, and molecular weight of the polymer, as well as the solvent used. The dielectric properties of the solution also play a key role (Kowalczyk et al., Biomacromolecules. 2008 Jul;9(7):2087-90; Thompson et al., J. Polymer. 2007;48:6913-6922; Mitchell and Sanders, J Biomed Mater Res A. 2006 Jul;78(1):110-20).

Another source of variation in electrospinning which is perhaps not well documented is that once the polymer is in solution it can change or degrade on storage and the same concentration of polymer does not always yield a solution with the same viscosity. Thus the molecular weight of the polymer will decrease rapidly over time (particularly the 50/50 PLGA). Therefore, fresh polymer is preferably used for spinning.

Preferably, the invention includes scaffolds formed from the co-electrospinning of said biodegradable polymer or co-polymer together with said NSAID. As used herein, the terms "co-electrospinning" and "co-electrospun" refer to the production of fibres comprising multiple components from a single solution comprising each of said components, for example a single solution comprising a biodegradable polymer or co-polymer together with said NSAID. Said polymer or copolymer and said NSAID are capable of each dissolving in a common solvent. Preferably said NSAID is directly spun into a scaffold comprising said polymer or copolymer.

As used herein, the term "biodegradable" refers to material or polymer that can be degraded, preferably adsorbed and degraded in a patient's body. The scaffold is biodegradable, i.e. is formed of biodegradable materials, such as biodegradable polymers naturally occurring biological material. Examples of suitable biodegradable materials include, but are not limited to collagen, poly(alpha esters) such as poly(lactate acid), poly(glycolic acid), polyorthoesters, polyanhydrides polyglycolic acid and polyglactin, and copolymers thereof.

Other suitable biodegradable polymers disclosed herein include cellulose ether, cellulose, cellulosic ester, fluorinated polyethylene, phenolic, poly-4- methylpentene, polyacrylonitrile, polyamide, polyamideimide, polyacrylate, polybenzoxazole, polycarbonate, polycyanoarylether, polyester, polyestercarbonate, polyether, polyetheretherketone, polyetherimide, polyetherketone, polyethersulfone, polyethylene, polyfluoroolefin, polyimide, polyolefin, polyoxadiazole, polyphenylene oxide, polyphenylene sulfide, polypropylene, polystyrene, polysulfide, polysulfone, polytetrafluoroethylene, polythioether, polytriazole, polyurethane, polyvinyl, polyvinylidene fluoride, regenerated cellulose, silicone, urea-formaldehyde, or copolymers or thereof.

Preferably the electrospun material is a polylactide, or a derivative thereof.

Preferably, the electrospun scaffold is functionalized, for example, by the addition of passive or active agents such as additional therapeutic or biological agents.

As used herein, the term "NSAID" and "non-steroidal anti-inflammatory compound" NSAIDs, pharmaceutically acceptable salts, complexes, esters, pharmaceutically acceptable isomers thereof, and crystalline forms thereof. The term relates to any NSAID capable of catalyzing the degradation of said biodegradable polymer or copolymer scaffold.

NASIDs are a well defined group of non-opioid analgesics characterised in that they are non-steroidal drugs which act as anti-inflammatory, analgesic and antipyretic agents.

Examples of NSAIDs include, but are not limited to NSAIDs which preferentially inhibit Cox-1, such as propionic acids, acetic acids, feamic acids, biphenylcarboxylic acids and oxicams.

Propionic acid NSAIDs include, but are not limited to, ibuprofen, naproxen, benoxaprofen, flurbiprofen, fenoprofen, fenbufen, ketoprofen, indoprofen, pirprofen, carprofen, oxaprozin, prapoprofen, miroprofen, tioxaprofen, suprofen, alminoprofen, tiaprofenic acid, fluprofen, and bucloxic acid. Preferably the propionic acid is ibuprofen.

Acetic acids include, but are not limited to, indomethacin, sulindac, tolmetin, zomepirac, diclofenac, fenchlofenac, alchlofenac, ibufenac, isoxepac, furofenac, tiopinac, zidometacin, acemetacin, fentiazac, clidanac and oxipinac.

Fenamic acids include but are not limited to, mefenamic acid, meclofenamic acid, flufenamic acid, niflumic acid and tolfenamic acid. Preferred members of the fenamic acid group include mefenamic acid and meclofenamic acid. Biphenylcarboxylic acids include, but are not limited to, diflunisal and flufenisal. Oxicams include meloxicam, piroxicam, sudoxican, isoxicam.

Alternatively, the NASID is a NSAID which preferentially inhibits Cox-2, such as lumiracoxib, celecoxib, rofecoxib, valdecoxib, parecoxib.

Alternatively, the NASID is a salicylate, such as aspirin.

As used herein the term "degradation" relates to the breakdown of the polymer structure of the scaffold. This breakdown of structural integrity is accompanied by the release from the scaffold of degradation products from the polymer and a reduction in the mechanical strength of the scaffold.

### Examples

### Materials and Methods

### 1. SCAFFOLD SYNTHESIS AND CHARACTERIZATION

### A. Polymers

For this study three poly(D,L-lactide-co-glycolide) (PLGA) copolymers with different ratios of lactide to glycolide 75:25 (Mw 66-107k), 50:50 (Mw 40-75k) and a 1:1 blend of these two were used. All the PLGA copolymers were purchased from Sigma Aldrich. The polymers were dissolved in dichloromethane (DCM) to form solutions of suitable viscosities for electrospinning; 75:25 and 50:50 to form 20% and 25% (wt/wt) solutions, respectively, and equal volumes of these solutions were used to form the blend. Ibuprofen powder (Sigma Aldrich) was added to the polymer solutions in DCM to form homogeneous mixtures (from 1-10% wt of Ibuprofen added relative to the wt of polymer).

### B. Electrospinning conditions

Polymer solutions were loaded into 2 ml syringes fitted with blunt tipped stainless steel needles with an internal diameter of 0.8 mm (I&J Fisnar Inc.). The solutions were delivered at a constant feed rate of 3.5 ml/hr using a programmable syringe pump (Aladdin 1000) and were electrospun horizontally with an accelerating voltage of 15 kV supplied by a high voltage power supply (Brandenburg, Alpha series III). Fibrous mats were collected on aluminium foil sheets (18 cm by 16 cm) wrapped around an earthed aluminium rotating collector (rotating at 300 rpm) 20 cm from the tip of the needle. Jet formation/stability was assisted by means of an aluminium focusing ring at a voltage of 15 kV, 5 mm behind the tip of the needle. Scaffolds were produced in an air conditioned room at 21 °C with a relative humidity of ∼30%, followed by drying under vacuum at room temperature for a minimum of 24 hours. To analyse the environmental effect (humidity and temperature) on scaffold reproducibility, scaffolds were occasionally spun in high humidity conditions (>85% relative humidity) and not dried under vacuum.

### C. Detection of Ibuprofen release from scaffolds by UV/Vis spectroscopy

The release of the Ibuprofen from the three scaffold types (each loaded with 10% Ibuprofen) was monitored using UV/Vis spectroscopy. A 25 mg piece of scaffold was cut from each scaffold produced and submerged in 3 ml of pure water. The intensity at 222 nm was measured (the λₘₐₓ of IBU) at various time points up to one week and the values were compared with a previously calculated Ibuprofen standard curve.

### D. In vitro degradation of scaffolds

Scaffolds with or without Ibuprofen were cut into 18 mm discs using a tissue punch and were sterilised by gently spraying them with 70% EtOH and left to dry in a sterile flow cabinet. Scaffolds were then immersed in 1 ml of Ringers solution with 100 IU/ml penicillin and 100 µg/ml streptomycin and incubated at 37°C. To test the effect of the pH on the degradation of the scaffold, they were placed in Ringers solution at 3 different pHs (6, 7.4 and 8.5). Samples of this solution were taken daily to monitor pH changes. Physical changes in the scaffold mats were also recorded daily by taking optical micrographs of the scaffolds.

### 2. CELL RESPONSE TO SCAFFOLDS IN 2D and 3D CULTURES

### A. Culture of normal human keratinocytes and fibroblasts

Normal human fibroblasts were isolated and cultured as described previously (Ralston *et al.,* 1997). Passage 3-7 fibroblasts were used. Keratinocytes were cultured in Dulbecco's modified Eagle's medium (DMEM) supplemented with 10% (v/v) foetal calf serum (FCS), 2 mM glutamine, 0.625µg/ml amphotericin B, 100IU/ml penicillin and 100µg/ml streptomycin. Preparation of 3D tissue engineered constructs either in Azowipe® scaffolds (nonwoven viscose rayon microfiber scaffolds bonded with styrene butadiene copolymer, Vernon-Carus, Chorley, UK) or PLGA 75:25 scaffolds was performed as previously described (Blackwood et al., Biomaterials. 2008; 29(21):3091-104).

### B. Detection of cellular metabolic activity via MTT-ESTA assay

The effect of Ibuprofen (sodium salt or isomer mixture; Sigma-Aldrich, UK) on cell metabolic activity, was studied by adding Ibuprofen directly onto the cells or by examining its release from scaffolds both in monolayers (2D) and in 3D scaffolds. For 2D analysis, 3x10⁴ HDF cells/well were cultured in 24 well plates until 80% confluent. For 3D analysis, 3x10⁵ HDF cells/well were cultured in 12 well plates in 2 layers of a viscose rayon scaffold for 48 hours. Scaffolds (with and without Ibuprofen) were placed above cultured cells using co-culture inserts so that scaffolds were immersed in the same media as the cells for 24 hours but were physically separated from the fibroblasts. Cellular metabolic activity (in 2D or 3D) was assessed using MTT (3-(4,5-dimethylthiazol-2-yl)-2,5 diphenyl tetrazolium bromide) (Sigma-Aldrich, St Louis MO). Briefly, cells were washed three times in PBS, and then incubated with MTT solution (0.5 mg/ml MTT in PBS, 1 and 2ml per well of 24 and 12 well plates, respectively) for 1 hour at 37 °C in a humidified incubator (5% CO₂/95% air). In healthy viable cells, MTT is reduced to a purple coloured formazan salt by the activity of the mitochondrial enzyme succinyl dehydrogenase. After 60 minutes, the solution was aspirated and the insoluble intracellular formazan product was solubilised and released from cells by adding iso-propanol (0.5 ml per well of 24 well plate or 1 ml / cm² cultured tissue) and incubated for 10 min. The optical density at 540 nm was then measured using a plate reading spectrophotometer.

### C. Visualisation of cells grown within scaffolds by staining for collagen production

To analyse the effect of environmental spinning conditions (humidity and temperature) on the physical structure of the scaffolds and on cell growth, 3D cultures on PLGA scaffolds electrospun under different conditions were tested for collagen deposition. Collagen deposition on scaffold fibres was analysed as described in Blackwood *et al.,* 2008. Briefly, constructs were grown for 7 days then washed 3 times in PBS and fixed in 4% formalin. Following 3 washes with PBS the constructs were stained for collagen deposition in a 0.1% solution of Sirius Red F3B (C.I. 35780, Direct Red 80, Sigma-Aldrich) in saturated picric acid for 18 hours. Afterwards, the constructs were washed with water until no more red colouring was eluted. Scaffolds stained with picrosirius red were then photographed with a digital camera.

### D. Investigation of the anti-inflammatory effect of Ibuprofen and Ibuprofen loaded scaffolds by immunolabelling of fibroblasts for the detection of NF-κB/p65 activation

LPS from E. coli serotype O111:B4 (Sigma-Aldrich) at 100ng/mL or TNF-α (Sigma-Aldrich) at 1000U/mL was used as a positive control for activation of NF-κB in fibroblasts. In preliminary experiments, Ibuprofen (10⁻³ and 10⁻⁴ M, sodium salt and isomer mixture; Sigma-Aldrich, UK) was used to downregulate NF-κB activation. Then the 10% Ibuprofen loaded PLGA scaffolds (75/25, 50/50 and a 1:1 blend of these two) were examined. Negative controls were produced by incubating cells in normal DMEM. All experiments were performed in triplicate.

Following the Ibuprofen treatments, monolayers were washed in PBS and the cells were fixed in 10% formalin (500µL per well) for 30 minutes. Cell monolayers were washed a further three times in PBS, prior to cell permeabilisation with 0.1 % Triton X100 for 20 minutes. Subsequently, cells were washed three times with PBS. Unreactive binding sites were blocked by adding a 5% dried milk powder solution to each well for 1 hour. A further three washes in PBS were performed prior to incubation with the first antibody solution. The first antibody (rabbit polyclonal antihuman IgG NF-κB p65 C-20, Santa Cruz Biotechnology Inc., CA) was added at a concentration of 1:100 in 1% PBS-dried milk powder. Cells were incubated in the antibody solution at 4°C for 18 hours. The cells were washed three more times in PBS. Afterwards, cells were incubated with biotinylated goat anti-rabbit (1:1000) (Vector Laboratories, CA) in 1% PBS-milk powder. The cells were then washed a further three times in PBS. Streptavidin-FITC (Vector Laboratories, CA) conjugate was then added to the wells at a concentration of 1:100 in PBS. A further three washes in PBS were performed. After this time, the fluorescent staining were visualised using an AXON image express system (Axon Instruments/Molecular Devices, Union City, CA). Briefly, fluorescence micrographs of immunolabelled samples were taken using epifluorescent illumination at λₑₓ 495 nm λₑₘ 515 nm (for FITC visualisation);λₑₓ 358 nm λₑₘ 461 nm (for DAPI visualisation). DAPI fluorescence was used to identify nuclear position. The boundary between nucleus and cytoplasm was delimited by analysis software (ImageXpress Console 1.0) using DAPI micrographs in a sample of at least 100 cellular counts per visual field. Within the defined region, the intensity of FITC (NF-κB/p65) labelling was used to calculate activation for each micrograph and sample. The calculation of NF-κB activation was performed manually according to intracellular position (as previously described in Moustafa et al., J Invest Dermatol. 2002 Dec;119(6):1244-53 and Canton et al, Tissue Eng. 2007;13(5):1013-24), where total nuclei localization indicates full activation of NF-κB and the absence of nuclear localization indicates inactive NF-κB. p65 labelling in both nuclei and cytosol was taken as partial activation. The analysis of the micrographs was done in triplicate.

For cells cultured in 3D scaffolds the methodologies used were as described in Sun et al, J Mater Sci Mater Med. 2004;15(7): 743-9. For investigation of fibroblast attachment and migration on Ibuprofen loaded scaffolds - this was examined by loading cells onto scaffolds containing 0, 2, 5 and 10% Ibuprofen and culturing cells for 7 days prior to assessment of cell viability using MTT ESTA. In these experiments some samples of scaffolds were washed by soaking in media for various periods of 1 to 48hrs prior to cell addition as indicated.

### E. Statistics

Student's unpaired t-test was used to assess the significance of differences between results.

### 3. ASPIRIN LOADED SCAFFOLDS

### A. Materials

Acetylsalicylic acid (Aspirin) and poly(D,L-lactide-co-glycolide) (75/25) were purchased from Sigma Aldrich and used as received.

### B. Electrospinning Aspirin loaded fibres

To a 20% wt solution of PLGA (75/25) in dichloromethane, 10% wt acetylsalicylic acid (relative to the polymer) was added and stirred until completely dissolved, giving a homogeneous solution. Electrospinning conditions were as for the Ibuprofen loaded PLGA 75/25 fibrous scaffolds, described above.

### C. Release of Aspirin

The release of Aspirin from the fibres was monitored using UV/Vis spectroscopy (intensity at 240 nm) at various time points over a one week period and compared with calibration curve values.

For each experiment, a section of scaffold (10 mg) was cut and submerged in pure water (3 ml). This corresponds to approximately 1.0 mg of Aspirin which is equivalent to a maximum calculated concentration of 1.68 mM.

### Results

### 1. SCAFFOLD SYNTHESIS AND CHARACTERISATION

### A. Development of reproducible spinning conditions for the production of electrospun material with Ibuprofen

PLGA scaffolds were electrospun from DCM solutions loaded with 1-10 % wt of Ibuprofen relative to the polymer to produce fibres in which the Ibuprofen was uniformly dispersed (see Fig 1A,D and F). The presence of the acid form of Ibuprofen did not significantly alter the appearance of the fibrous scaffolds as shown in Fig 1. The acid form was used as the sodium salt of Ibuprofen was insoluble in solvents suitable for processing the polymer. The latter was found to result in a heterogeneous dispersion of the drug in the spinning solution and on spinning, the drug had a tendency to come to the surface of the fibres due to the electrostatic forces involved in the process (see Fig 1 B).

Fibres produced in this way have been shown to have a rapid burst release when submerged in aqueous media (Taepaiboon, et al, Nanotechnology. 2006;17:2317-232). Accordingly to achieve a more sustained release, homogeneous solutions of the polymer and Ibuprofen isomer mixture (acid form) were prepared in dichloromethane (DCM) for all subsequent studies.

The concentrations of the various polymers and the electrospinning parameters were as previously described by us (Blackwood et al., Biomaterials. 2008; 29(21):3091-104). 1-10% wt of Ibuprofen (relative to the polymer) was added and each solution was thoroughly dissolved prior to spinning. For the 1:1 'blend' of polymers, equal volumes of the solutions of PLGA 50/50 (with Ibuprofen) and 75/25 (with Ibuprofen) were mixed.

The temperature and humidity of the environment in which the electrospinning was performed strongly influenced the physical properties of the scaffold. Due to the rapid solvent evaporation of the spinning solution as the ejected fibre dries, it becomes cooler than its surroundings and water vapour condenses upon it. Spinning in humid conditions produced scaffolds which cells contracted independent of the cell type grown on them or the presence or absence of serum in the medium (Fig. 2A). Here it can be seen that after only a few days in culture with cells (fibroblast, keratinocytes and co-cultures of both), the scaffolds underwent obvious physical modifications (contraction, loss of thickness and the fibres merged together to give a porous film like morphology). However, vacuum drying the scaffolds immediately after spinning in order to thoroughly remove any surface water produced scaffolds that were much more resistant to cell contraction as seen in Fig 2B.

### B. Ibuprofen release from the scaffolds and in vitro degradation of scaffolds

The release of the Ibuprofen from the three scaffold types (each containing 10% Ibuprofen) was monitored using UV/Vis spectrometry. For each experiment, a piece of scaffold of 25 mg was cut and submerged in 3 ml of pure water. This corresponded to around 2.5 mg of Ibuprofen per sample, which is equivalent to a maximum calculated concentration of 4.1 mM, 3.9 mM and 3.2 mM for the 50/50, 75/25 and blend respectively. The release of Ibuprofen from the samples was monitored with UV/Vis spectroscopy at 222 nm (the λₘₐₓ of Ibuprofen) at various time points and compared with the calibration curve values. The release profiles for the three scaffolds are shown in Figure 3 a and b.

All the scaffolds showed similar behaviour, with an initial rapid release of drug during the first 8 hours (as shown on an expanded scale in Fig 3b). This initial rapid release of Ibuprofen was then followed by a sustained release over the following six days (as shown in Figure 3a), by which point none of the scaffolds had totally released the drug; in fact, the concentrations of drug in solution were around an order of magnitude lower than the calculated values for the total amount of drug present. Accordingly scaffolds appear to have lost approximately one tenth of their loaded Ibuprofen over 7 days.

The higher the percentage of glycolide to lactide in the copolymer, the faster the breakdown of the scaffolds both *in vivo* and *in vitro* (Blackwood et al., Biomaterials. 2008; 29(21):3091-104). The 50/50 scaffold broke down very rapidly (within a week *in vitro*) compared to those composed of 75% lactide and 25% glycolide which took around 3 months. Thus initially work focused on the 50/50 scaffold as the best candidate for an Ibuprofen releasing dressing which would degrade into the wound bed. However, the hydrolytic degradation of PLGA is acid catalysed and it was believed that the presence of Ibuprofen (acidic) within the scaffold could have an effect on the breakdown rate of the scaffold.

Accordingly the effect of including Ibuprofen in the scaffolds on the *in vitro* degradation of scaffolds with different ratios of glycolide (50/50, 75/25 and a 1:1 blend of 50/50 and 75/25) was investigated. Also as it has been reported that the pH in non-healing wounds can range from acidic to basic (Dissemond et al., Hautarzt. 2003 Oct;54(10):959-65), these variations in pH were mimicked *in vitro* in Ringer's solution to pH 6, 7.4 and 8.5. In Figure 4 we show optical micrographs of some of the 75:25 scaffold fibres at times 0, 1 and 6 days with and without Ibuprofen. Table 2 summarises the degradation data from these experiments.

To begin with, all the scaffolds looked similar (with fibres ranging from 2-5 µm) whether scaffolds contained Ibuprofen or not (Fig 4). However, by day 6 the scaffolds carrying Ibuprofen had lost all structural integrity. In contrast the PLGA scaffolds (irrespective of the glycolide content) without Ibuprofen maintained similar appearances to those seen at day 0 (Fig 4 and Table 1).

**Table 1. pH^{†} of the Ringers solution around the scaffold**

| | **Initial pH** | **pH 6** | | **pH 7.4** | | **pH8.5** | |
|---|---|---|---|---|---|---|---|
| | **Ibuprofen** | - | + | - | + | - | + |
| **Day1** | **75:25** | 4.7 | 4.4 | 5.5 | 5.0 | 5.0 | 5.6 |
| | **50:50** | 4.4 | 4.4 | 5.0 | 4.5 | 4.4 | 5.6 |
| | **Blend** | 4.1 | 3.2 | 5.0 | 5.3 | 4.7 | 5.6 |
| **Day 6** | **75:25** | 3.6 | 2.6 | 4.1 | 3.5 | 3.5 | 4.7 |
| | **50:50** | 4.1 | 4.1 | 4.4 | 3.5 | 4.1 | 4.7 |
| | **Blend** | 3.5 | 2.6 | 3.4 | 4.4 | 2.9 | 4.7 |

| | | | | | | | |
|---|---|---|---|---|---|---|---|
| †Scaffolds (with or without ibuprofen; 50:50, 75:25, and the blend) were cut into 18 mm diameter discs using a tissue punch. Scaffolds were immersed in 1 ml of Ringers solution with antibiotics (1.25µg/ml amphotericin B, 200 IU/ml penicillin and 200µg/ml streptomycin) and incubated at 37°C. The pH of the solution around the scaffold was monitored at days 0 (initial pH)-not shown and days 1 and 6. | | | | | | | |

This degradation was also independent of the initial pH of the Ringer's solution which did not change significantly from the pH of 4-5 seen in the Ringer's solution around the scaffold at day 1. This did not change significantly over the 6 days of the experiment (Table 2).

As summarised in Table 2 the content of glycolide was a key factor in speeding up the rate of degradation of the fibres. The PLGA 50/50 scaffold had the fastest rate of degradation with Ibuprofen present, indeed by day one the physical structure of the Ibuprofen 50/50 scaffold showed some merging of fibres (as shown in Fig 4C and summarised in Table 2).

**Table 2 Effect of Ibuprfoen and pH on in vitro degradation of PLGA scaffolds**

| | | **Extent of visible degradation** | | | | | |
|---|---|---|---|---|---|---|---|
| | | **Control scaffolds** | | | **+ Ibuprofen scaffolds** | | |
| **Day** | **Polymer** | **pH 6** | **pH 7.4** | **pH 8.5** | **pH 6** | **pH 7.4** | **pH 8.5** |
| 0 | 50:50 | 0 | 0 | 0 | 0 | 0 | 0 |
| | 75:25 | 0 | 0 | 0 | 0 | 0 | 0 |
| | 1:1 of above | 0 | 0 | 0 | 0 | 0 | 0 |
| 1 | 50:50 | 0 | 0 | 0 | + | + | + |
| | 75:25 | 0 | 0 | 0 | + | + | + |
| | 1:1 of above | 0 | 0 | 0 | + | + | + |
| 6 | 50:50 | 0 | 0 | 0 | ++ | ++ | ++ |
| | 75:25 | 0 | 0 | 0 | ++ | ++ | ++ |
| | 1:1 of above | 0 | 0 | 0 | ++ | ++ | ++ |
| Visible degradation scored as none=0, some + or extensive ++ | | | | | | | |

### 2. CELL RESPONSE TO SCAFFOLDS

### A. Cytotoxic and anti-inflammatory responses of cells to Ibuprofen

Initially it was necessary to determine which concentrations of Ibuprofen would be anti-inflammatory without being cytotoxic for cells. Accordingly both the Ibuprofen sodium salt and an isomer mixture of Ibuprofen (acid form) were tested for their effects on viability of fibroblasts (results summarised in Table 3). Both forms of Ibuprofen performed similarly, with cells tolerating concentrations of Ibuprofen up to 10⁻³ M (p<0.05) but showing a cytotoxic response at concentrations of 10⁻² M. For future studies the concentrations of Ibuprofen investigated did not exceed 10⁻³M.

**Table 3 . Effect of Nalbu and Ibuprofen isomer mixture on HDF viability (MTT-ESTA Assay)**

| | | | | | | |
|---|---|---|---|---|---|---|
| The effect of 24 h exposure to Ibuprofen on human fibroblast viability was assessed using the MTT-ESTA assay. Results shown are means +- SEM of triplicate wells. Values differing from controls are indicated as *p <0.05 | | | | | | |

| Treatment | Ibuprofen Sodium Salt | | | Ibuprofen isomer mixture | | |
|---|---|---|---|---|---|---|
| | %Viability | SEM | pvalue | %Viability | SEM | Pvalue |
| Control | **100** | **16.43** | | **100** | **16.43** | |
| 10⁻⁶ | **102.17** | **17.71** | | **109.43** | **15.53** | |
| 10⁻⁵ | **100.43** | **18.55** | | **111.80** | **13.19** | |
| 10⁻⁴ | **98.21** | **14.04** | | **108.56** | **15.71** | |
| 10⁻³ | **90.33** | **11.70** | | **101.11** | **18.30** | |
| 10⁻² | **38.25** | **5.79** | **P<0.05** | **7.20** | **2.47** | **P<0.05** |

We next examined the effects of Ibuprofen on LPS and TNF induced NF-κB activation in fibroblasts (Fig.5 and 6). Concentrations of Ibuprofen of 10⁻⁴ and 10⁻³M - both the Ibuprofen salt and the Ibuprofen isomer mix -were capable of significantly reducing the response to LPS and TNF-κα as illustrated in Figure 5 and quantitative data is shown in Figure 6. In these experiments LPS and TNF-α produced 80% activation of NF-κB.

### B. Cytotoxic and anti-inflammatory responses of cells to Ibuprofen scaffolds.

The next stage of experimentation was to place PLLA-PGA scaffolds both unloaded and loaded with Ibuprofen above cultures of fibroblasts in 3D scaffolds. The effect of the PLGA scaffolds on cell viability and on the response of cells to pro-inflammatory stimulation was then investigated. The loaded scaffolds were examined in two ways. Some were deliberately left unwashed so that any initial burst release of Ibuprofen would be obtained in these experiments. Others were vigorously washed in 70% EtOH and then washed three times in PBS to remove any loosely bound Ibuprofen.

There was no significant effect of the washed and unwashed scaffolds on the viability of the fibroblast monolayers placed under these scaffolds irrespective of whether the scaffolds were loaded with 10% Ibuprofen or not.

Any initial burst release of Ibuprofen from the scaffolds does not appear to be high enough to adversely affect adjacent cellular viability to any significant degree. Calculations of the maximum release of Ibuprofen into the one ml of media over 24 hours gave a theoretical maximum of 0.4 mM (as derived from Section 2 above assuming approximately 10% release of the 10% Ibuprofen loaded into the scaffolds.

With respect to the anti-inflammatory effects of Ibuprofen incubation of 2D monolayers of fibroblasts with the scaffolds containing Ibuprofen significantly reduced LPS stimulated NF-κB translocation in all cases (p<0.05) (Fig 7). The inhibition observed with the three different scaffolds was not significantly different, and comparable to that observed when cells were incubated with an isomer mix of Ibuprofen at 10⁻⁴M as shown in Figure 6. Therefore it appears that 10% Ibuprofen loaded into scaffolds was released from the scaffolds in concentrations appropriate to inhibit inflammatory signalling in fibroblasts.

The effect of the scaffolds containing Ibuprofen on cells cultured in 3D was examined. Example images of the appearance of these cells in 3D are shown in Figure 8. While it is technically challenging to estimate NF-κB activation using the p65 translocation in 3D, two points could be noted from this study. Firstly there was less activation of the cells with LPS in 3D than 2D (as has been reported from this and other laboratories cells often cope better with insults when in 3D rather than 2D (Sun et al, J Mater Sci Mater Med. 2004;15(7): 743-9) and secondly the 10% Ibuprofen scaffolds were capable of reducing the LPS activation of NF-κB in these cells in 3D.

### C. Ability of Ibuprofen loaded scaffolds to support cell attachment and proliferation

Finally a comparison of fibroblast attachment and proliferation on the scaffolds showed that fibroblasts attached and proliferated on scaffolds loaded with 1,2 and 5% wt of Ibuprofen as well as on non-loaded scaffolds (Figure 9a). However loading of scaffolds with 10% Ibuprofen did reduce initial cell attachment but this could be partially (see Figure 9a) or fully (see Figure 9b) restored to control levels by washing the scaffolds for 24 hours (consistent with scaffolds releasing some of their Ibuprofen).

This shows that the Ibuprofen loaded fibres remain capable of provided a substrate for these cells to attach and migrate on and is confirmed by the results shown in table 4.

**Table 4. Effect of scaffolds loaded with Ibuprofen on fibroblast viability in 3D culture**

| | **Scaffold treatment** | **Scaffold composition** | **Normalised % viability** |
|---|---|---|---|
| **Non loaded control scaffolds** | **Washed** | **Blend** | 94.0±1.1 |
| | | **75/25** | 94.4±4.5 |
| | | **50/50** | 73.8±7.2 |
| | **Unwashed** | **Blend** | 74.8±7.0 |
| | | **75/25** | 70.6±9.2 |
| | | **50/50** | 74.2±11.9 |
| **Ibuprofen loaded scaffolds** | **Washed** | **Blend** | 108.5±4.5 |
| | | **75/25** | 112.4±0.2 |
| | | **50/50** | 82.1±11.3 |
| | **Unwashed** | **Blend** | 85.5±10.4 |
| | | **75/25** | 98.5±7.2 |
| | | **50/50** | 101.1±7.2 |

Fibroblasts were cultured in 3D cultures (in triplicate) and the effects of scaffolds loaded or unloaded with Ibuprofen were examined on these cultures for 24 hours assessing cell viability MTT ESTA assay. Results shown are means ± SEM of triplicates of cell viability relative to the control cultures not exposed to scaffolds.

The scaffold compositions were poly(D,L-lactide-co-glycolide) (PLGA) copolymers were electrospun to produce the scaffolds with different ratios of lactide to glycolide [75:25 (Mw 66-107k), 50:50 (Mw 40-75k) and a 1:1 blend of these two.

Scaffold treatments were as follows. All scaffolds with or without Ibuprofen were initially sterilised using 70%EtOH. The scaffolds were then washed in cell medium for a few seconds prior to placing over the 3D cultures. For Ibuprofen scaffolds referred to as washed scaffolds these were washed in PBS for 1 hour to allow for any initial Ibuprofen release and then washed briefly in cell medium for a few seconds prior to placing over the 3D scaffolds containing fibroblasts.

### 3. ASPIRIN LOADED SCAFFOLDS

### A. Results

The total concentration of Aspirin in the 10 mg sample of scaffold (in 3 ml water) was 1.68 mM and over one week only 37% of this had been released. As with the Ibuprofen loaded scaffolds, there was an initial rapid release in the first 8 hours or so followed by a more sustained, gradual release over the following 7 days.

### B. Discussion

The release profile of Aspirin from the PLGA fibrous scaffolds was very similar to that seen for the Ibuprofen loaded scaffolds, with an initial rapid release over the first 8 hours. After the initial burst, a more sustained release is observed as the fibres begin to degrade but in each case scaffolds had only released a percentage (approximately 10% for Ibuprofen and 37% for Aspirin) after 7 days submerged in water, as illustrated in figure 10.

## Claims

1. An electrospun scaffold, comprising a biodegradable polymer or co-polymer and a nonsteroidal anti-inflammatory drug (NSAID), wherein said polymer or copolymer and said NSAID are capable of each dissolving in a common solvent and said NSAID is capable of catalyzing the degradation of the polymer or copolymer, wherein:
a) said biodegradable polymer is a collagen, a poly alpha ester, a polyorthoester or a polyanhydride or a copolymer thereof, and
b) said NSAID is acetylsalicylic acid or a propionic NSAID.

2. An electrospun scaffold according to claim 1, wherein said polymer is polylactate acid or polyglycolic acid.

3. An electrospun scaffold according to claim 1, wherein said NSAID is [2-(4-Isobutylphenyl)propionic acid].

4. An electrospun scaffold according to claim 1, wherein said copolymer is a copolymer of polylactate acid and polyglycolic acid.

5. An electrospun scaffold according to claim 4, wherein said copolymer is a poly(D,L-lactic-co-glycolide), and optionally wherein said poly(D,L-lactic-co-glycolide) has a ratio of 75:25 lactide to glycolide or said poly(D,L-lactic-co-glycolide) has a ratio of 50:50 lactide to glycolide.

6. An electrospun scaffold according to any one of claims 1 to 5, wherein said scaffold comprises from about 5% to about 30% by weight of NSAID, from about 10% to 20% by weight of NSAID, or about 10% by weight of NSAID.

7. A wound dressing comprising the electrospun scaffold of any one of claims 1 to 6.

8. An electrospun scaffold, said scaffold comprising a biodegradable polymer or co-polymer and a nonsteroidal anti-inflammatory drug (NSAID), for use as a medicament, wherein said polymer or copolymer and said NSAID are capable of each dissolving in a common solvent and said NSAID is capable of catalyzing the degradation of the polymer or copolymer, wherein:
a) said biodegradable polymer is a collagen, a poly alpha ester, a polyorthoester or a polyanhydride or a copolymer thereof, and
b) said NSAID is acetylsalicylic acid or a propionic NSAID.

9. An electrospun scaffold, said scaffold comprising a biodegradable polymer or co-polymer and a nonsteroidal anti-inflammatory drug (NSAID) for use in the treatment of a wound, preferably a chronic wound or an acute wound, wherein said polymer or copolymer and said NSAID are capable of each dissolving in a common solvent and said NSAID is capable of catalyzing the degradation of the polymer or copolymer, wherein:
a) said biodegradable polymer is a collagen, a poly alpha ester, a polyorthoester or a polyanhydride or a copolymer thereof, and
b) said NSAID is acetylsalicylic acid or a propionic NSAID.

10. An electrospun scaffold comprising a biodegradable polymer or co-polymer and a nonsteroidal anti-inflammatory drug (NSAID), for use in the treatment of pain or inflammation, wherein said polymer or copolymer and said NSAID are capable of each dissolving in a common solvent and said NSAID is capable of catalyzing the degradation of the polymer or copolymer, wherein:
a) said biodegradable polymer is a collagen, a poly alpha ester, a polyorthoester or a polyanhydride or a copolymer thereof, and
b) said NSAID is acetylsalicylic acid or a propionic NSAID.

11. A method of producing an electrospun scaffold for use in wound healing comprising:
i) dissolving a polymer or co-polymer and a nonsteroidal anti-inflammatory drug (NSAID) in a common solvent, wherein said NSAID is capable of catalysing the degradation of the polymer or copolymer and
a) said biodegradable polymer is a collagen, a poly alpha ester, a polyorthoester or a polyanhydride or a copolymer thereof, and
b) said NSAID is acetylsalicylic acid or a propionic NSAID; and
ii) electrospinning the resulting solution of step i).

12. An electrospun scaffold produced in accordance with the method of claim 11.

13. Use of a nonsteroidal anti-inflammatory drug (NSAID) to catalyse the degradation of an electrospun scaffold comprising a polymer or co-polymer, wherein said polymer or copolymer and said NSAID are capable of each dissolving in a common solvent and said NSAID is capable of catalyzing the degradation of the polymer or copolymer, and wherein
a) said biodegradable polymer is a collagen, a poly alpha ester, a polyorthoester or a polyanhydride or a copolymer thereof, and
b) said NSAID is acetylsalicylic acid or a propionic NSAID, optionally wherein said NSAID is electrospun together with said polymer or copolymer.

## Patentansprüche

1. Ein elektrogesponnenes Gerüst, umfassend ein biologisch abbaubares Polymer oder Copolymer und ein nichtsteroidales entzündungshemmendes Arzneimittel (NSAID), wobei jenes Polymer oder Copolymer und jenes NSAID in der Lage sind sich jeweils in einem gemeinsamen Lösungsmittel zu lösen und jenes NSAID in der Lage ist den Abbau des Polymers oder Copolymers zu katalysieren, wobei:
a) jenes biologisch abbaubare Polymer ein Collagen, ein Poly-Alpha-Ester, ein Polyorthoester oder ein Polyanhydrid oder ein Copolymer davon ist, und
b) jenes NSAID Acetylsalicylsäure oder ein propionisches NSAID ist.

2. Ein elektrogesponnenes Gerüst entsprechend Anspruch 1, wobei jenes Polymer Polymilchsäure oder Polyglycolsäure ist.

3. Ein elektrogesponnenes Gerüst entsprechend Anspruch 1, wobei jenes NSAID [2-(4-Isobutylphenyl)propionsäure] ist.

4. Ein elektrogesponnenes Gerüst entsprechend Anspruch 1, wobei jenes Copolymer ein Copolymer aus Polymilchsäure und Polyglycolsäure ist.

5. Ein elektrogesponnenes Gerüst entsprechend Anspruch 4, wobei jenes Copolymer ein Poly(D,L-Lactid-co-Glycolid) ist, und wobei optional jenes Poly(D,L-Lactid-co-Glycolid) ein Verhältnis von 75:25 Lactid zu Glycolid hat oder jenes Poly(D,L-Lactid-co-Glycolid) ein Verhältnis von 50:50 Lactid zu Glycolid hat.

6. Ein elektrogesponnenes Gerüst entsprechend irgendeinem der Ansprüche 1 bis 5, wobei jenes Gerüst von etwa 5 Gewichts-% bis etwa 30 Gewichts-% von NSAID, von etwa 10 Gewichts-% bis 20 Gewichts-% von NSAID oder etwa 10 Gewichts-% von NSAID umfasst.

7. Eine Wundauflage, umfassend das elektrogesponnene Gerüst von einem der Ansprüche 1 bis 6.

8. Ein elektrogesponnenes Gerüst, jenes Gerüst umfassend ein biologisch abbaubares Polymer oder Copolymer und ein nichtsteroidales entzündungshemmendes Arzneimittel (NSAID), zur Verwendung als Medikament, wobei jenes Polymer oder Copolymer und jenes NSAID in der Lage sind sich jeweils in einem gemeinsamen Lösungsmittel zu lösen und jenes NSAID in der Lage ist den Abbau des Polymers oder Copolymers zu katalysieren, wobei:
a) jenes biologisch abbaubare Polymer ein Collagen, ein Poly-Alpha-Ester, ein Polyorthoester oder ein Polyanhydrid oder ein Copolymer davon ist, und
b) jenes NSAID Acetylsalicylsäure oder ein propionisches NSAID ist.

9. Ein elektrogesponnenes Gerüst, jenes Gerüst umfassend ein biologisch abbaubares Polymer oder Copolymer und ein nichtsteroidales entzündungshemmendes Arzneimittel (NSAID), zur Verwendung bei der Behandlung einer Wunde, vorzugsweise einer chronischen Wunde oder einer akuten Wunde, wobei jenes Polymer oder Copolymer und jenes NSAID in der Lage sind sich jeweils in einem gemeinsamen Lösungsmittel zu lösen und jenes NSAID in der Lage ist den Abbau des Polymers oder Copolymers zu katalysieren, wobei:
a) jenes biologisch abbaubare Polymer ein Collagen, ein Poly-Alpha-Ester, ein Polyorthoester oder ein Polyanhydrid oder ein Copolymer davon ist, und
b) jenes NSAID Acetylsalicylsäure oder ein propionisches NSAID ist.

10. Ein elektrogesponnenes Gerüst umfassend ein biologisch abbaubares Polymer oder Copolymer und ein nichtsteroidales entzündungshemmendes Arzneimittel (NSAID), zur Verwendung bei der Behandlung von Schmerz oder Entzündung, wobei jenes Polymer oder Copolymer und jenes NSAID in der Lage sind sich jeweils in einem gemeinsamen Lösungsmittel zu lösen und jenes NSAID in der Lage ist den Abbau des Polymers oder Copolymers zu katalysieren, wobei:
a) jenes biologisch abbaubare Polymer ein Collagen, ein Poly-Alpha-Ester, ein Polyorthoester oder ein Polyanhydrid oder ein Copolymer davon ist, und
b) jenes NSAID Acetylsalicylsäure oder ein propionisches NSAID ist.

11. Ein Verfahren zur Herstellung eines elektrogesponnenen Gerüsts zur Verwendung in der Wundheilung, umfassend:
i) Lösen eines Polymers oder Copolymers und eines nichtsteroidalen entzündungshemmenden Arzneimittels (NSAID) in einem gemeinsamen Lösungsmittel, wobei jenes NSAID in der Lage ist den Abbau des Polymers oder Copolymers zu katalysieren und
a) jenes biologisch abbaubare Polymer ein Collagen, ein Poly-Alpha-Ester, ein Polyorthoester oder ein Polyanhydrid oder ein Copolymer davon ist, und
b) jenes NSAID Acetylsalicylsäure oder ein propionisches NSAID ist; und
ii) Elektrospinnen der resultierenden Lösung aus Schritt i).

12. Ein elektrogesponnenes Gerüst, das entsprechend dem Verfahren von Anspruch 11 hergestellt wurde.

13. Verwendung eines nichtsteroidalen entzündungshemmenden Arzneimittels (NSAID) um den Abbau eines elektrogesponnenen Gerüsts zu katalysieren, das ein Polymer oder ein Copolymer umfasst, wobei jenes Polymer oder Copolymer und jenes NSAID in der Lage sind sich jeweils in einem gemeinsamen Lösungsmittel zu lösen und jenes NSAID in der Lage ist den Abbau des Polymers oder Copolymers zu katalysieren, wobei:
a) jenes biologisch abbaubare Polymer ein Collagen, ein Poly-Alpha-Ester, ein Polyorthoester oder ein Polyanhydrid oder ein Copolymer davon ist, und
b) jenes NSAID Acetylsalicylsäure oder ein propionisches NSAID ist,
wobei optional jenes NSAID zusammen mit jenem Polymer oder Copolymer elektrogesponnen wurde.

## Revendications

1. Échafaudage électrofilé comprenant un polymère ou un copolymère biodégradable et un médicament anti-inflammatoire non stéroïdien (AINS), dans lequel ledit polymère ou copolymère et ledit AINS peuvent chacun se dissoudre dans un solvant commun et ledit AINS peut catalyser la dégradation du polymère ou du copolymère, dans lequel :
a) ledit polymère biodégradable est un collagène, un polyalphaester, un polyorthoester ou un polyanhydride ou un copolymère de ceux-ci, et
b) ledit AINS est l'acide acétylsalicylique ou un AINS propionique.

2. Échafaudage électrofilé selon la revendication 1, dans lequel ledit polymère est l'acide polylactate ou un l'acide polyglycolique.

3. Échafaudage électrofilé selon la revendication 1, dans lequel ledit AINS est l'[acide 2-(4-isobutylphényl)propionique].

4. Échafaudage électrofilé selon la revendication 1, dans lequel ledit copolymère est un copolymère de l'acide polylactate et de l'acide polyglycolique.

5. Échafaudage électrofilé selon la revendication 4, dans lequel ledit copolymère est un poly(D,L-lactique-co-glycolide), et éventuellement dans lequel ledit poly(D,L-lactique-co-glycolide) présente un rapport lactide au glycolide de 75:25 ou ledit poly(D,L-lactique-co-glycolide) présente un rapport lactide à glycolide de 50:50.

6. Échafaudage électrofilé selon l'une quelconque des revendications 1 à 5, dans lequel ledit échafaudage comprend d'environ 5 % à environ 30 % en poids de l'AINS, d'environ 10 % à 20 % en poids de l'AINS ou environ 10 % en poids de l'AINS.

7. Pansement pour plaie comprenant l'échafaudage électrofilé de l'une quelconque des revendications 1 à 6.

8. Échafaudage électrofilé, ledit échafaudage comprenant un polymère ou un copolymère biodégradable et un médicament anti-inflammatoire non stéroïdien (AINS), pour utilisation comme médicament, dans lequel ledit polymère ou copolymère et ledit AINS peuvent chacun se dissoudre dans un solvant commun et ledit AINS peut catalyser la dégradation du polymère ou du copolymère, dans lequel :
a) ledit polymère biodégradable est un collagène, un polyalphaester, un polyorthoester ou un polyanhydride ou un copolymère de ceux-ci, et
b) ledit AINS est l'acide acétylsalicylique ou un AINS propionique.

9. Échafaudage électrofilé, ledit échafaudage comprenant un polymère ou un copolymère biodégradable et un médicament anti-inflammatoire non stéroïdien (AINS), pour utilisation dans le traitement d'une plaie, de préférence une plaie chronique ou une plaie aiguë, dans lequel ledit polymère ou copolymère et ledit AINS peuvent chacun se dissoudre dans un solvant commun et ledit AINS peut catalyser la dégradation du polymère ou du copolymère, dans lequel :
a) ledit polymère biodégradable est un collagène, un polyalphaester, un polyorthoester ou un polyanhydride ou un copolymère de ceux-ci, et
b) ledit AINS est l'acide acétylsalicylique ou un AINS propionique.

10. Échafaudage électrofilé comprenant un polymère ou un copolymère biodégradable et un médicament anti-inflammatoire non stéroïdien (AINS), pour utilisation dans le traitement d'une douleur ou d'une inflammation, dans lequel ledit polymère ou copolymère et ledit AINS peuvent chacun se dissoudre dans un solvant commun et ledit AINS peut catalyser la dégradation du polymère ou du copolymère, dans lequel :
a) ledit polymère biodégradable est un collagène, un polyalphaester, un polyorthoester ou un polyanhydride ou un copolymère de ceux-ci, et
b) ledit AINS est l'acide acétylsalicylique ou un AINS propionique.

11. Procédé de production d'un échafaudage électrofilé pour utilisation dans la guérison de plaies, comprenant :
i) la dissolution d'un polymère ou d'un copolymère et d'un médicament anti-inflammatoire non stéroïdien (AINS) dans un solvant commun, dans lequel ledit AINS peut catalyser la dégradation du polymère ou du copolymère et
a) ledit polymère biodégradable est un collagène, un polyalphaester, un polyorthoester ou un polyanhydride ou un copolymère de ceux-ci, et
b) ledit AINS est l'acide acétylsalicylique ou un AINS propionique ; et
ii) l'électrofilage de la solution résultante de l'étape i).

12. Échafaudage électrofilé produit conformément au procédé de la revendication 11.

13. Utilisation d'un médicament anti-inflammatoire non stéroïdien (AINS) pour catalyser la dégradation d'un échafaudage électrofilé qui comprend un polymère ou un copolymère, dans lequel ledit polymère ou copolymère et ledit AINS peuvent chacun se dissoudre dans un solvant commun et ledit AINS peut catalyser la dégradation du polymère ou du copolymère, et dans lequel
a) ledit polymère biodégradable est un collagène, un polyalphaester, un polyorthoester ou un polyanhydride ou un copolymère de ceux-ci, et
b) ledit AINS est l'acide acétylsalicylique ou un AINS propionique, éventuellement dans lequel ledit AINS est électrofilé avec ledit polymère ou copolymère.
